# EUROPEAN PATENT SPECIFICATION

(11) **EP 4 125 910 B1**
(45) Date of publication and mention of the grant of the patent: **12.11.2025**
(21) Application number: 21776098.2
(22) Date of filing: 08.01.2021
(51) Int. Cl.: A61K 31/551, A61K 31/506, A61K 31/519, A61K 39/395, A61P 25/28

(54) **RHO KINASE INHIBITORS FOR TREATING FRONTOTEMPORAL DEMENTIA**
RHO-KINASEHEMMERN ZUR BEHANDLUNG VON FRONTOTEMPORALER DEMENZ
INHIBITEURS DE LA KINASE ASSOCIÉE À RHO POUR TRAITER LA DÉMENCE FRONTO-TEMPORALE

(30) Priority: 25.03.2020 US 202062994478 P; 16.04.2020 US 202063011008 P
(43) Date of publication of application: 08.02.2023
(73) Proprietor: Woolsey Pharmaceuticals, Inc., St. Petersburg, FL 33702 (US)
(72) Inventor: MACALLISTER, Thomas, Arlington, Virginia 22201 (US); JACOBSON, Sven, New York, New York 10005 (US)
(74) Representative: Canet, Denis
(86) International application number: PCT/US2021/012582
(87) International publication number: WO 2021/194607

(56) References cited:
- WO-A1-2021/162808
- US-A1- 2002 119 140
- US-A1- 2010 113 551
- US-A1- 2011 294 789
- US-A1- 2012 010 196
- US-A1- 2015 031 683
- US-A1- 2018 193 386
- US-B2- 8 796 293
- GENTRY E. G. ET AL: "Rho Kinase Inhibition as a Therapeutic for Progressive Supranuclear Palsy and Corticobasal Degeneration", vol. 36, no. 4, 27 January 2016 (2016-01-27), US, pages 1316 - 1323, XP055935778, ISSN: 0270-6474, Retrieved from the Internet <URL:https://www.jneurosci.org/content/jneuro/36/4/1316.full.pdf> DOI: 10.1523/JNEUROSCI.2336-15.2016
- GENTRY ERIK G ET AL: "Rho kinase ii (ROCK2) inhibition reduces tau levels via autophagy in neurons", ALZHEIMER'S & DEMENTIA, vol. 11, no. 7, 20 July 2015 (2015-07-20), XP029354809, ISSN: 1552-5260, DOI: 10.1016/J.JALZ.2015.07.198
- ANONYMOUS: "Frontotemporal dementia", WIKIPEDIA, 22 January 2020 (2020-01-22), pages 1 - 11, XP055864089, Retrieved from the Internet <URL:https://en.wikipedia.org/w/index.php?title=Frontotemporal_dementia&oldid=937025283> [retrieved on 20210307]

## Description

### Cross-Reference to Related Applications

This application claims priority to U.S. Provisional application no. 62/994,478 filed on March 25, 2020, and to U.S. Provisional Patent application 63/011,008 filed on April 16, 2020.

### Background of the Invention

Frontotemporal dementia (FTD) is a progressive neurodegenerative syndrome involving atrophy of the prefrontal and anterior temporal cortices. It is sometimes conflated with frontotemporal lobar degeneration (FTLD), but FTLD refers to a broader group of syndromes that include FTD. FTD can manifest as three clinically recognized subtypes, one behavioral and two language variants, each associated with specific areas of neurodegeneration in the brain that can be detected by neuroimaging.

Accounting for about two-thirds of FTD cases, behavioral variant FTD (bvFTD or bvFTLD) shows predominant prefrontal neurodegeneration, with neuropsychological revealing deficits on frontal or executive tasks, but relative sparing of episodic memory and visuospatial abilities. Patients with bvFTD present clinically with marked changes in behavior and personality, displaying a combination of disinhibition, apathy, lack of emotional concern, hyperorality, and stereotypic behavior.

The two language variants are progressive non-fluent (agrammatic) aphasia (PNFA or navPPA) and semantic dementia (SD), which is also known as the semantic variant of progressive primary aphasia (svPPA). In contrast to bvFTD, atrophy in SD/svPPA affects mainly the anterior temporal lobes and in PNFA it affects the left perisylvian region. (Mesulam 2009)

In addition to FTD, FTLD further encompasses frontotemporal dementia with motor neuron disease (FTD-MND), progressive supranuclear palsy syndrome (PSP-S) and corticobasal syndrome (CBS). All of these conditions are characterized by degeneration of the prefrontal and/or anterior temporal cortex, but have different clinical manifestations, genetic backgrounds and likely different underlying pathophysiologies.

FTD occurs in 5-15% of patients with dementia and occurs with equal frequency in both sexes. The age of onset is usually between 45 and 65 years though it may range anywhere from 21 to 81 years. FTD, unlike Alzheimer's disease has a strong genetic basis, with family history in 40-50% of cases. The gene encoding tau is often linked to FTD.

Layered onto a complicated genetic story, the neuropathology of FTD is diverse, though constituted primarily by tau or TAR DNA-binding protein 43 (TDP-43) depositions. The current understanding of FTD lacks any clear-cut relationship between clinical phenotype and neuropathological features. Only in certain cases of monogenetic disease is there any predictability in terms of neuropathology. In particular, GRN mutations and C9orf72 expansions are associated with TDP neuropathology while MAPT mutations are associated with Tau.

Kamei (1996a and 1996b) reported on using fasudil in two patients with wandering due to vascular dementia. One patient was diagnosed with Binswanger-type cerebral infarction, confirmed by MRI imaging. The other patient was diagnosed with sequelae of cerebral bleeding and multiple lacunar infarctions, confirmed by MRI. Despite the preliminary results for wandering in a few patients with subcortical vascular dementia, there is no evidence that this observation, even if confirmed by a clinical study, could be extrapolated to treatment of underlying dementias in the cortical regions of the brain.

Erik G. Gentry et al.; The Journal of Neuroscience, 2016, pages 1316-1323 highlights the Rho kinases as rational therapeutic targets to combat tau accumulation in progressive supranuclear palsy and corticobasal degeneration.

There are no approved drugs for the management of FTD and so there is a need to find effective agents that is met by the present invention.

### Summary of the Invention

Note that the references to the methods of treatment by therapy in this description are to be interpreted as references to compounds, pharmaceutical compositions and medicaments of the present invention for use in those methods.

The invention relates to methods of treating frontotemporal dementia that generally comprise administering to a patient suffering from frontotemporal dementia a pharmacologically effective amount of a rho kinase inhibitor.

The rho kinase inhibitor is fasudil or a pharmaceutically acceptable salt thereof. The frontotemporaldementia is the behavioral variant frontotemporal dementia (bvFTD), the progressive non-fluent (agrammatic) aphasia (PNFA) or the semantic dementia (SD),

In a preferred embodiment, the patient is suffering from the behavioral variant frontotemporal dementia.

According to the invention, the patient has no neuromuscular symptoms and/or has not been diagnosed with amyotrophic lateral sclerosis, corticobasal degeneration, Parkinson's Disease or progressive supranuclear palsy.

Preferred embodiments contemplate the treatment of FTD patients with aggregates of tau or TDP-43.

In one embodiment, the FTD patient treated with fasudil has a defect in the *C9ORF72* gene. In a specific embodiment, the defect is a hexanucleotide repeat expansion in a 5' non-coding region of the *C9ORF72* gene.

In a further embodiment, the FTD patient treated with fasudil has a defect in the *VCP* gene (valosin-containing protein), the *CHMP2B* (charged multivesicular body protein 2B, the *TARDP* (TAR DNA binding protein), the *TREM2* (triggering receptor expressed on myeloid cells 2), the *UBQLN2* (ubiquilin 2), the *SQSTM1* (sequestosome 1) gene, the *GBA* (β-glucocerebrosidase) gene, the *ABCA7* ( ATP binding cassette subfamily A member 7) gene, the *PARK7* (Parkinsonism-associated deglycase 7) gene, the *SORL1* (sortilin related receptor 1) gene, the *LRRK2* (leucine rich repeat kinase 2) gene, or the *ALS2* (alsin Rho guanine nucleotide exchange factor) gene, wherein the patient does not have another neurodegenerative disease.

In another embodiment, the FTD patient treated with fasudil has a defect in the *MAPT* (tau) or *GRN* (progranulin).

In a further embodiment the FTD patient treated with fasudil has aggregation of tau, FUS (fused in sarcoma) or TDP-43, but does not have ALS.

In a further embodiment, the patient to be treated with fasudil has sporadic FTD.

In one embodiment of the invention, the FTD patient treated with fasudil exhibits delayed progression of disease. In a specific embodiment, the patient treated exhibits delayed memory loss.

In on embodiment of the invention, the patient treated wherein the treatment eliminates use of antipsychotic medications (e.g., aripiprazole, clozapine, haloperidol, olanzapine, quetiapine, risperidone and ziprasidone). antidepressant to combat behavioral symptoms.

In a further embodiment, the patient treated with fasudil is not also being treated with active agents including mood stabilizers, benzodiazepines, antipsychotics, anti-agitation drugs, or sleep aids. In a specific embodiment, the patient treated with fasudil is not being treated with risperidone, aripiprazole, quetiapine, carbamazepine, gabapentin, prazosin, trazodone or lorazepam.

In another embodiment, the patient treated with fasudil is also treated with an anti-depressant such as trazodone, and SSRIs such as citalopram or escitalopram, paroxetine, fluoxetine, or sertraline.

In a further embodiment the patient treated with fasudil eliminates the need for co-treatment with an antidepressant.

In one embodiment, the patient treated exhibits improved behavioral symptoms of FTD wherein the symptoms include loss of empathy, shallow affect, repetitive and/or obsessive or compulsive behavior, aberrant or excessive eating, hypochondriasis, social misconduct, impaired judgment, and excess sleeping.

In another embodiment, the patient treated exhibits decreased or delayed loss of speech or hyperorality.

In further embodiment, treatment with fasudil reduces cerebral atrophy in the frontal and anterior temporal lobes of the brain.

In one embodiment, the patient treated with fasudil is age 50 or younger.

In another embodiment, the patient treated has FTD with a 3R tauopathy neuropathology.

The methods of the invention are preferably accomplished using fasudil administered orally in a total daily dose of between 70 mg and 140 mg.

### Detailed Description of the Invention

The present invention relates to the treatment of FTLD and more specifically to the FTD as defined in claim 1.

Preferably, the invention relates to the treatment of the bvFTD, resulting in improvements in prefrontal atrophy or degeneration, alleviation of deficits in frontal or executive tasks, improvements in behavior and personality changes as well as enhancements of memory and/or cognition.

The most common form of FTD, bvFTD is frequently referred to simply as frontotemporal dementia or Pick's disease. As used herein, FTD refers to bvFTD, along with the two language variants, SD and PNFA.

FTLD is defined by the focal neurodegeneration in the frontal and anterior temporal lobes of the brain. FTLD spectrum disorders include FTD-MND, PSP-S and CBS. FTD, however, is distinct from the other FTLD spectrum disorders in that it is not fundamentally a neuromuscular condition and patients can present exclusively with central nervous system deficiencies without motor deficiencies. Thus, while ALS shares some common pathology with FTD, the present invention does not address motor symptoms and is, therefore, not generally applicable to ALS treatment.

A variety of genetic mutations have been associated with FTD, the most common being those in the microtubule-associated protein tau (MAPT) gene, the granulin (GRN) gene, or the expansion on chromosome 9 open reading frame 72 (C9orf72) gene. Genes less commonly associated with FTD include valosin-containing protein (VCP) mutations, which are linked to a specific condition called inclusion body myopathy with Paget disease of the bone and FTD (IBMPFD), charged multivesicular body protein 2B (CHMP2B), which is involved in the endosomal-lysosomal pathway, fused in sarcoma (FUS), TAR DNA-binding protein (TARDBP), sequestosome 1 (SQSTM1), TANK-binding kinase 1 (TBK1), and ubiquilin 2 (UBQLN2).

FTD, and FTLD generally, are pathologically associated with protein aggregopathy, wherein mutated or misfolded versions of normal proteins for inclusions in the brain. The most common protein aggregates are tau, TDP-43, and FUS (rarely). Tau is a neuronal protein involved in stabilizing axonal microtubules. Phosphorylation of tau inhibits its function and promotes protein aggregation (Amano 2003). TDP-43 is a protein involved in transcriptional activation and DNA repair. Protein aggregates of TDP-43 contain ubiquinated, hyperphosphorylated TDP-43 protein. FUS is also involved in transcriptional regulation and DNA repair.

FTD and FTLD generally can be pathologically subdivided into tau-positive and tau-negative types and the majority of patients will be tau-positive. Tau exists in 6 isoforms that can be roughly divided into two groups based on the inclusion or exclusion one of four repeated microtubule-binding domains (MTBD), resulting in so-called 3R (three repeat) tau or 4R (four repeat) tau. Tauopathies are classified by the relative predominance of 3R versus 4R tau isoforms found in cytoplasmic inclusions. Those composed predominantly of 3R tau result in "3R tauopathies" and those with predominantly 4R tau result in "4R tauopathies." Tau normally exists in an equal ratio of 3R:4R tau in non-disease states and also can exist in equal ratios in diseased states, such as AD.

Among the FTLD spectrum, PSP-S and CBS are known to be 4R tauopathies, with the tau deposits consisting predominantly of the 4-repeat version of tau. Pick's disease or bvFTD is considered to be a 3R tauopathy because the characteristic Pick bodies comprise predominantly 3R tau, though on autopsy some bvFTD patients have been shown to have other neuropathologies.

For non-tau pathologies, hyperphosphorylated, ubiquitinated TDP-43 is the most common protein found in inclusions.

### ROCK Inhibitors

The inventive methods contemplate the administration of a rho kinase (ROCK) inhibitor in the treatment of a disease or condition defined in the claims.

Two mammalian ROCK homologs are known, ROCK1 (aka ROKβ, Rho-kinase β, or p160ROCK) and ROCK2 (aka ROKα) (Nakagawa 1996). In humans, the genes for both ROCK1 and ROCK2 are located on chromosome 18. The two ROCK isoforms share 64% identity in their primary amino acid sequence, whereas the homology in the kinase domain is even higher (92%) (Jacobs 2006; Yamaguchi 2006). Both ROCK isoforms are serine/threonine kinases and have a similar structure.

A large number of pharmacological ROCK inhibitors are known (Feng, LoGrasso, Defert, & Li, 2015). Isoquinoline derivatives are a preferred class of ROCK inhibitors. The isoquinoline derivative fasudil was the first small molecule ROCK inhibitor developed by Asahi Chemical Industry (Tokyo, Japan). The characteristic chemical structure of fasudil consists of an isoquinoline ring, connected via a sulphonyl group to a homopiperazine ring. Fasudil is a potent inhibitor of both ROCK isoforms. *In vivo,* fasudil is subjected to hepatic metabolism to its active metabolite hydroxyfasudil (aka, M3). Other examples of isoquinoline derived ROCK inhibitors include dimethylfasudil and ripasudil.

Other non-claimed ROCK inhibitors are based on based on 4-aminopyridine structures. These were first developed by Yoshitomi Pharmaceutical (Uehata et al., 1997) and are exemplified by Y-27632. Still other preferred ROCK inhibitors include indazole, pyrimidine, pyrrolopyridine, pyrazole, benzimidazole, benzothiazole, benzathiophene, benzamide, aminofurazane, quinazoline, and boron derivatives (Feng et al., 2015). Some exemplary ROCK inhibitors are shown below:

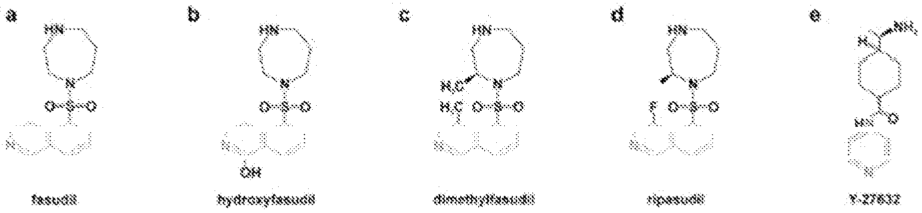

ROCK inhibitors may have more selective activity for either ROCK1 or ROCK2 and will usually have varying levels of activity on PKA, PKG, PKC, and MLCK. Some ROCK inhibitors may be highly specific for ROCK1 or ROCK2 and have much lower activity against PKA, PKG, PKC, and MLCK.

According to the present invention, the ROCK inhibitor is fasudil. Fasudil may be exist as a free base or salt and may be in the form of a hydrate, such as a hemihydrate.

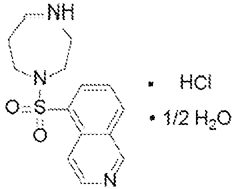

### Hexahydro-1-(5-isoquinolinesulfonyl)-1H-1,4-diazepine monohydrochloride hemihydrate

Fasudil is a selective inhibitor of protein kinases, such as ROCK, PKC and MLCK and treatment results in a potent relaxation of vascular smooth muscle, resulting in enhanced blood flow (Shibuya 2001). A particularly important mediator of vasospasm, ROCK induces vasoconstriction by phosphorylating the myosin-binding subunit of myosin light chain (MLC) phosphatase, thus decreasing MLC phosphatase activity and enhancing vascular smooth muscle contraction. Moreover, there is evidence that fasudil increases endothelial nitric oxide synthase (eNOS) expression by stabilizing eNOS mRNA, which contributes to an increase in the level of the potent vasodilator nitric oxide (NO), thereby enhancing vasodilation (Chen 2013).

Fasudil has a short half-life of about 25 minutes, but it is substantially converted *in vivo* to its 1-hydroxy (M3) metabolite. M3 has similar effects to its fasudil parent molecule, with slightly enhanced activity and a half-life of about 8 hours (Shibuya 2001). Thus, M3 is likely responsible for the bulk of the *in vivo* pharmacological activity of the molecule. In other words, fasudil is essentially a prodrug of M3 and methods involving administration of fasudil should be seen as equivalent to administering M3, just as other prodrugs of M3 should be considered to be methods of administering M3. M3 exists as two tautomers, depicted below:

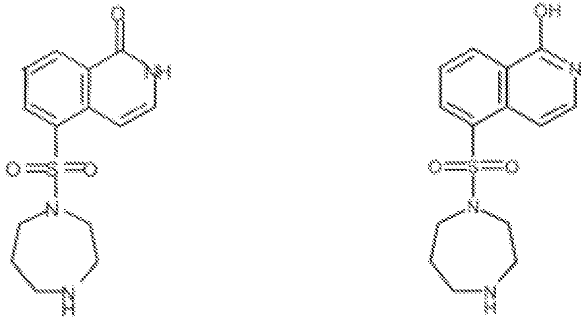

The ROCK inhibitor used in the invention, fasudil, include pharmaceutically acceptable salts and hydrates. Salts that may be formed via reaction with inorganic and organic acid. Those inorganic and organic acids are included as following: hydrochloric acid, hydrobromide acid, hydriodic acid, sulphuric acid, nitric acid, phosphoric acid, acetic acid, maleic acid, maleic acid, maleic acid, oxalic acid, oxalic acid, tartaric acid, malic acid, mandelic acid, trifluoroacetic acid, pantothenic acid, methane sulfonic acid, or para-toluenesulfonic acid.

### Pharmaceutical Compositions

Pharmaceutical compositions of ROCK inhibitors are generally oral and may be in the form of tablets or capsules and may be immediate-release formulations or may be controlled-or extended-release formulations, which may contain pharmaceutically acceptable excipients, such as corn starch, mannitol, povidone, magnesium stearate, talc, cellulose, methylcellulose, carboxymethylcellulose and similar substances. A pharmaceutical composition comprising the ROCK inhibitor and/or a salt thereof may comprise one or more pharmaceutically acceptable excipients, which are known in the art. Formulations include oral films, orally disintegrating tablets, effervescent tablets and granules or beads that can be sprinkled on food or mixed with liquid as a slurry or poured directly into the mouth to be washed down.

Pharmaceutical compositions containing ROCK inhibitors, salts and hydrates thereof can be prepared by any method known in the art of pharmaceutics. In general, such preparatory methods include the steps of bringing the ROCK inhibitor or a pharmaceutically acceptable salt thereof into association with a carrier or excipient, and/or one or more other accessory ingredients, and then, if necessary and/or desirable, shaping, and/or packaging the product into a desired single- or multi-dose unit.

Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered. The composition used in accordance with the methods of the present invention may comprise between 0.001% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils. Excipients such as cocoa butter and suppository waxes, coloring agents, coating agents, sweetening, flavoring, and perfuming agents may also be present in the composition.

In certain embodiments, the pharmaceutical composition used in the methods of the present invention may comprise a diluent. Exemplary diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

In certain embodiments, the pharmaceutical composition used in the methods of the present invention may comprise a granulating and/or dispersing agent. Exemplary granulating and/or dispersing agents include potato starch, corn starch, tapioca starch, sodium starch glycolate, clays, alginic acid, guar gum, citrus pulp, agar, bentonite, cellulose, and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscannellose), methylcellulose, pregelatinized starch (starch 1500), microcrystalline starch, water insoluble starch, calcium carboxymethyl cellulose, magnesium aluminum silicate (VEEGUM), sodium lauryl sulfate, quaternary ammonium compounds, and mixtures thereof.

In certain embodiments, the pharmaceutical composition used in the methods of the present invention may comprise a binding agent. Exemplary binding agents include starch (e.g., cornstarch and starch paste), gelatin, sugars (e.g., sucrose, glucose, dextrose, dextrin, molasses, lactose, lactitol, mannitol, etc.), natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (VEEGUM.RTM.), and larch arabogalactan), alginates, polyethylene oxide, polyethylene glycol, inorganic calcium salts, silicic acid, polymethacrylates, waxes, water, alcohol, and/or mixtures thereof.

In certain embodiments, the pharmaceutical composition used in the methods of the present invention may comprise a preservative. Exemplary preservatives include antioxidants, chelating agents, antimicrobial preservatives, antifungal preservatives, antiprotozoan preservatives, alcohol preservatives, acidic preservatives, and other preservatives. In certain embodiments, the preservative is an antioxidant. In other embodiments, the preservative is a chelating agent.

In certain embodiments, the pharmaceutical composition used in the methods of the present invention may comprise an antioxidant. Exemplary antioxidants include alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite.

In certain embodiments, the pharmaceutical composition used in the methods of the present invention may comprise a chelating agent. Exemplary chelating agents include ethylenediaminetetraacetic acid (EDTA) and salts and hydrates thereof (e.g., sodium edetate, disodium edetate, trisodium edetate, calcium disodium edetate, dipotassium edetate, and the like), citric acid and salts and hydrates thereof (e.g., citric acid monohydrate), fumaric acid and salts and hydrates thereof, malic acid and salts and hydrates thereof, phosphoric acid and salts and hydrates thereof, and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal.

In certain embodiments, the pharmaceutical composition may comprise a buffering agent together with the ROCK inhibitor or the salt thereof. Exemplary buffering agents include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

In certain embodiments, the pharmaceutical composition used in the methods of the present invention may comprise a lubricating agent. Exemplary lubricating agents include magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

In other embodiments, the pharmaceutical composition of containing the ROCK inhibitor or salt thereof will be administered as a liquid dosage form. Liquid dosage forms for oral and parenteral administration include pharmaceutically acceptable emulsions, microemulsions, solutions, suspensions, syrups, and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (e.g., cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents. In certain embodiments for parenteral administration, the conjugates of the invention are mixed with solubilizing agents such as Cremophor^{™}, alcohols, oils, modified oils, glycols, polysorbates, cyclodextrins, polymers, and mixtures thereof.

Solid dosage forms for oral administration include capsules, tablets, pills, powders, and granules. In such solid dosage forms, the active ingredient is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may include a buffering agent.

Some compositions of the invention relate to extended- or controlled-release formulations. These may be, for example, diffusion-controlled products, dissolution-controlled products, erosion products, osmotic pump systems or ionic resin systems. Diffusion-controlled products comprise a water-insoluble polymer which controls the flow of water and the subsequent egress of dissolved drug from the dosage from. Dissolution-controlled products control the rate of dissolution of the drug by using a polymer that slowly solubilizes or by microencapsulation of the drug - using varying thicknesses to control release. Erosion products control release of drug by the erosion rate of a carrier matrix. Osmotic pump systems release a drug based on the constant inflow of water across a semi permeable membrane into a reservoir which contains an osmotic agent. Ion exchange resins can be used to bind drugs such that, when ingested, the release of drug is determined by the ionic environment within the gastrointestinal tract.

### Treatable Patients

Patients treatable according to the invention will have one or more symptoms of FTLD that may be confirmed by imaging. The patients have FTD as defined in claim 1. The FTD may be one of the two language variants - progressive non-fluent (agrammatic) aphasia (PNFA) and semantic dementia (SD), which is also known as the semantic variant of progressive primary aphasia (svPPA) - but it is preferably the behavioral variant (bvFTD). An overview of the affected brain regions (detectable by imaging) and the primary deficits that are used in diagnosing FTD are shown in Table 1.

**Table 1: Characteristics of FTD Subtypes**

| **Subtype** | **Region of Brain Atrophy** | **Presentation** |
|---|---|---|
| bvFTLD (behavioral) | prefrontal | Deficits in frontal or executive tasks; marked behavior and personality changes |
| SD (speech) | anterior temporal | Deficits in comprehension and naming; motor speech is spared |
| PNFA (speech) | perisylvian | Deficits in motor speech and grammatical sentence construction; intact comprehension |

Imaging is as useful tool in diagnosing dementia, in particular computerized tomography (CT), magnetic resonance imaging (MRI) and positron emission spectroscopy (PET). Neural degeneration results in brain atrophy and this can be detected and quantified. Automated tools are increasingly available that can perform these functions.

Fluorodeoxyglucose (FDG) PET scans measure glucose use in the brain. Glucose, a type of sugar, is the primary source of energy for cells. Studies show that people with dementia often have abnormal patterns of decreased glucose use in specific areas of the brain. An FDG PET scan can show a pattern that may support a diagnosis of a specific cause of dementia.

Evaluation of CSF biomarkers (e.g., β-amyloid 42) can also be used to differentiate from Alzheimer's-associated dementia. For example, a decreased cerebrospinal fluid (CSF) p-Tau₁₈₁ to total tau ratio (p/t-tau) is a biomarker for frontotemporal lobar degeneration with TDP43 inclusions (FTLD-TDP). CSF neurofilament light chain (NfL) levels are increased in FTLD-TDP and are correlated with disease severity and the degree of brain atrophy and with survival and can be used to measure prognosis.

The hallmarks of bvFTD are personality changes, apathy, and a progressive decline in socially appropriate behavior, judgment, self-control, and empathy. Unlike in Alzheimer's disease, memory is usually relatively spared in bvFTD. People with bvFTD typically do not recognize the changes in their own behavior, or exhibit awareness or concern for the effect their behavior has on the people around them. They may also exhibit repetitive behavior, including lapping or pacing. Methods according to the invention involve treating patients with bvFTD using an amount of the ROCK inhibitor sufficient to positively affect one or more of the following symptoms: personality changes, apathy, social behavior, judgment, self-control, repetitive behavior, compulsive behavior and empathy. Treatment according to the invention typically begins after symptoms are evident, even though many cases have an apparent genetic basis. Currently, no prognostic test exists and so treating asymptomatic patients is not preferred. Patients with SD develop progressive comprehension deficits and naming errors, but speech production is otherwise spared. PNFA is characterized by loss of motor speech fluency and agrammatism, with relatively intact language comprehension. The distinctions between the three FTD clinical subtypes tends to blur with the progression of the disease, likely reflecting the spreading of atrophy to broader areas of the frontal and temporal regions. But immunohistochemical investigations have revealed that ubiquitin/TAR DNA binding protein-43 (TDP-43) positive and tau negative pathology, mostly FTLD with ubiquitin-positive inclusions (FTLD-U) type 3, accounts for 90% of PNFA cases, while the remaining 10% may be caused by tauopathy. Treatment of patients with SD or PNFA are among preferred methods of the invention.

While the clinical symptoms of bvFTD can overlap with FTLD spectrum disorders, including progressive supranuclear palsy (PSP), corticobasal syndrome (CBS) and Amyotrophic lateral sclerosis-FTD spectrum disorder (ALS-FTSD), an important distinction is that the dominant symptoms are motor dysfunction in those disorders. In contrast, the three forms of FTD are not neuromuscular conditions and so do not exhibit the motor dysfunction. Thus, certain preferred embodiments of the invention directed to pure degermation of the frontotemporal area of the brain, the presence of motor dysfunction is excluded and/or patients do not have PSP, CBS or ALD-FTSD. In embodiments of the invention that do include the broader FTLD spectrum, the treatment of female patients as a subset is particularly preferred, while the treatment of males is also contemplated. In some variants the hormonal milieu affects the nature and progression of the disease and so treatment of females is different from treating males.

Historically, most FTD trials have used cognitive or functional measures designed for Alzheimer's disease, such as the MMSE, Clinical Dementia Rating (CDR), the Alzheimer's Disease Assessment Scale-Cognitive Subscale (ADAS-Cog) and the Alzheimer's Disease Cooperative Study-Clinical Global Impression of Change (ADCS-CGIC), including variants thereof. However, these were designed principally to detect the impaired executive function and memory characteristic of AD, rather than the social and emotional deficits more characteristic of FTD. Thus, particularly appropriate tools for measuring FTD are the Social Cognition and Emotional Assessment (SEA), the Frontotemporal Dementia Rating Scale (FRS), the Frontal Systems Behavior Scale (FrSbe) and the FTLD Clinical Dementia Rating scale. Improvements in each of the scales enumerated herein are contemplated with the inventive treatments.

The SEA is composed of five subtests, each assessing a specific orbitofrontal-related function: a test of identification of facial emotions, a reversal/extinction task, a behavioral control task, a theory of mind test, and an apathy scale. The maximum score is 55. A cut-off score of 39.4 is proposed to separate normal controls from bvFTLD (Funkiewiez et al., 2012).

The Frontotemporal Dementia Rating Scale (Mioshi 2010) evaluates six severity stages based upon a 30-item questionnaire (very mild to profound). Greater levels of impairment are seen in the bvFTLD than in the language variant or semantic variant. The score ranges from -6.66 to 5.39. When converted to percentages, 0-2% is profound impairment; 3-12% is very severe impairment; 13-40 is severe impairment; 41-79 is moderate impairment; 80-96 is mild impairment; and 97-100 is very mild impairment.

The FrSBe is a 46-item rating scale, with three subscales: Apathy (14 items), Disinhibition (15 items) and Executive dysfunction (17 items). Item content of the Apathy scale samples "problems with initiation, psychomotor retardation, spontaneity, drive, persistence, loss of energy and interest, lack of concern about self/care, and/or blunted affective expression". (Stout 2003). The Disinhibition subscale items assess problems with inhibitory control of actions and emotions, including impulsivity, hyperactivity, social inappropriateness, emotional liability, explosiveness, irritability. Problem areas addressed in the Executive dysfunction subscale include "sustained attention, working memory, organization, planning, future orientation, sequencing, problem solving, insight, mental flexibility, self-monitoring of ongoing behavior, and/or the ability to benefit from feedback or modify behavior following errors. The Self-rating and Family forms have identical items, phrased as appropriate. Items are rated in a 5-point scale: 1 (almost never), 2 (seldom), 3 (sometimes), 4 (frequently), 5 (almost always). Four scores are obtained: Total, Apathy, Disinhibition and Executive. Scores greater than T = 65 are considered clinically significant.

The Clinical Dementia Rating scale (CDR) is a 5-point scale used to characterize six domains of cognitive and functional performance: Memory, Orientation, Judgment & Problem Solving, Community Affairs, Home & Hobbies, and Personal Care. (Morris 1997). CDR-0 connotes no cognitive impairment, and then the remaining four points are for various stages of dementia: 0.5 = questionable, or very mild dementia; 1 = mild; 2 = moderate; and 3 = severe.

The defining cognitive decline in SD appears to be a deterioration of semantic knowledge. Many of the testing procedures for assessing semantic knowledge in SD rely on visual stimuli (e.g., picture naming) or the ability to understand words with strong visual associations. The impairment is greater for less familiar words and objects. (Bonner 2010)

In PNFA speech rate (words per minute) is about one third to one half that of normal, healthy individuals and the patients make grammatical errors.

Patients treatable according to the invention will typically score poorly on cognitive scales, such as the mini mental state exam (MMSE), however patients can also be treated before symptom onset if they have been identified at high risk of FTD and/or have a familial variant. A threshold of ≤ 23 on the MMSE is set for dementia, with score of ≤15 Representing severe dementia. The MMSE, is described fully in Folstein (Folstein 1975 and Rovner 1987). Once the MMSE falls below 15, the Severe Impairment Battery (SIB) is a useful assessment too.

The MMSE, is described fully in Folstein (1975, 1987 and 2007). Generally, an MMSE score of 24-30 indicates no cognitive impairment, a score of 18-23 indicates mild cognitive impairment and 0-17 indicates severe cognitive impairment.

Recent studies have used the Neuropsychiatric Inventory (Connor 2008) and particularly the apathy/indifference domain score to assess efficacy in FTD studies. The NIH EXAMINER battery, which looks at executive function (Kramer 2014) has also been used, as has the appetite and eating habit questionnaire (APEHQ) (Ahmed 2016). Improvements in each of these scales is envisioned to result from the inventive methods.

Electrophysiological measures, such as facial electromyography, transcranial magnetic stimulation, can reveal impaired cortical inhibition and plasticity in people with FTD, in some cases before symptoms develop (see Burrell et al., 2011; Benussi et al., 2017).

bvFLTD patients will generally show improvements in deficits in frontal or executive tasks and marked behavior and personality changes and/or a slowing in decline vs. control patients following commencement of treatment. Patients with bvFTD may improve, or deteriorate less than a control, on any one or more of the following: marked changes in behavior and personality, disinhibition, apathy, lack of emotional concern, hyperorality, and stereotypic behavior. In addition, patients with bvFTLD can seem impervious to pain, heat, and cold, and are less averse to bad odors (although they do not have anosia). SD patients will generally show improvements in deficits in comprehension and naming and/or a slowing in decline vs. control following commencement of treatment. Patients with SD with semantic dementia appear more sensitive to pain, heat, and cold, unlike with bvFTD. The Frontotemporal Dementia Rating Scale also can be used to evaluate SD. (Mioshi 2010).

PNFA patients will generally show improvements in deficits in motor speech and grammatical sentence construction and/or a slowing in decline vs. control following commencement of treatment. The Frontotemporal Dementia Rating Scale also can be used to evaluate SD. (Mioshi 2010).

In one embodiment, treatment with fasudil increase the number of words used by SD and/or PNFA patients by at least 10%, 15%, 20% 40% or at 50%. In a specific embodiment, treatment with fasudil increase the number of correct words used by SD and/or PNFA patients by at least 10%, 15%, 20% 40% or at 50%.

In one embodiment, treatment with fasudil increase the number of words used by SD and/or PNFA patients by at least 10%, 15%, 20% 40% or at 50%.

For all types of FTD, treatment with fasudil reduces the build-up of TDP-43 or tau. In a specific embodiment, the reduction is in the left temporal lobe.

In one embodiment, treatment with fasudil results in a mean increase in MMSE score of greater than 3 points. In a specific embodiment, the increase with fasudil results in an MMSE score of ≥ 23.

### Methods of Treatment

In accordance with the treatment methods of the present invention, an effective amount of the ROCK inhibitor or a pharmaceutically acceptable salt thereof for administration one or more times a day may comprise from about 10 mg to about 1000 mg. Fasudil hydrochloride hemihydrate, for example, is suitably administered in a daily amount of about 10 mg to about 500 mg, about 10 mg to about 400 mg, about 10 mg to about 200 mg, about 10 mg to about 100 mg, about 20 mg to about 10 mg. One preferred dosing regimen involves the treatment with 20, 30 or 40 mg of Fasudil hydrochloride hemihydrate three times per day using an immediate-release formulation, for a total daily dose of 60 - 120 mg. Most preferred dosing exceeds a daily dose of 60 mg, with most preferred ranges for daily dosing being 70 mg to 120 mg administered in three equal amounts during the day. Other preferred daily doses will range from 90 mg to 120 mg per day or 80 mg to 140 mg per day. A further dosing regimen involves the treatment with, 30 to 60 mg of Fasudil hydrochloride hemihydrate only two times per day using an immediate-release formulation, for a total daily dose of 60 - 120 mg. Based on ROCK inhibitory activity, one skilled in the art can readily extrapolate the provided dosing ranges for fasudil to other ROCK inhibitors. A preferred embodiment is 45 mg of fasudil hydrochloride hemihydrate two times per day using an immediate-release formulation.

Certain patient sub-populations, such as renally impaired patients and/or older patients (e.g., 65 or older) may need lower doses or extended release formulations instead of immediate release formulations. Fasudil hydrochloride hemihydrate may have higher steady-state concentrations when given at usual doses to patients with renal disease and lower doses to lower the Cmax or delay the time to Cmax (increase the Tmax) may be required.

Kidney function is most often assessed using serum (and/or urine) creatinine. Creatinine is a breakdown product of creatine phosphate in muscle cells and it is produced at a constant rate. It is excreted by the kidneys unchanged, principally through glomerular filtration. Accordingly, elevated serum creatinine is a marker for kidney dysfunction and it is used to estimate glomerular filtration rate.

Normal levels of creatinine in the blood are approximately 0.6 to 1.2 mg/dL in adult males and 0.5 to 1.1 mg/dL in adult females. When creatinine levels exceed these figures, the subject has renal dysfunction, and is, therefore, treatable according to the invention. Mild renal impairment/dysfunction occurs in the range of 1.2 mg/dL to 1.5 mg/dL. Moderate renal impairment/dysfunction is considered to occur at creatinine levels exceeding 1.5 mg/dL. Severe renal impairment, which includes what is considered to be renal failure, is defined as a serum creatinine level of ≥ 2.0 mg/dL or the use of renal replacement therapy (such as dialysis). Treating subjects with mild, moderate and severe renal impairment is specifically contemplated.

As indicated, creatinine levels are considered to be a surrogate for glomerular filtration rate and serum creatinine levels alone may be used to estimate glomerular filtration rate using the Cockroft-Gault equation.

Generally, creatinine clearance of less than 60 mL/min (corresponding roughly to creatinine of > 1.2 mg/dL) is considered moderate renal dysfunction. A glomerular filtration rate below 40 mL/min (corresponding approximately to creatinine levels exceeding 1.5 mg/dL) or especially 30 mL/min is considered severe renal dysfunction.

In general, creatinine clearance (estimated glomerular filtration rate) may be derived directly from serum creatinine using the Cockroft - Gault equation: creatinine clearance = (((140 - age in years) × (wt in kg)) × 1.23)/(serum creatinine in µmol/L)

For women the result of the calculation is multiplied by 0.85.

Empirically measured creatinine clearance may also be used directly as an estimate of glomerular filtration rate by looking at serum creatinine and urine creatinine levels. Specifically, urine is collected over 24 hours and the following equation is applied to ascertain creatinine clearance: Creatinine Clearance (mL/min) = Urine Creatinine Concentration (mg/mL) * 24 hour urine volume (mL)/Plasma Creatinine Concentration (mg/mL) * 24 hour * 60 minutes

In one embodiment, dose of fasudil for mild to moderate renal impairment is reduced to 50-80 mg per day. In another embodiment, the dose of fasudil is not reduced but is administered one time per day in an extended release dosage form.

In another embodiment, the dose is not reduced for mild to moderate renal impairment.

In one embodiment, the dose of fasudil is reduced to 30-45 for severe renal impairment. In another embodiment, the dose of fasudil is not reduced but is instead administered one time per day in an extended release dosage form.

In a further embodiment, the dose is reduced where serum creatinine (SCr) >2 and/or an increase in SCr > 1.5x from baseline, and/or a decrease in eGFR >25% from baseline.

Patient size is an important factor to consider when using creatinine-based estimates of renal function. The units of drug clearance are volume/time (mL/min), whereas the units of estimated GFR for chronic renal disease are volume/time/standard size (mL/min/1.73m2). Generally doses may be adjusted down (e.g., 40-50 mg per day) for smaller patients and up for larger (e.g., 120 mg per day) for obese patients. A smaller male would be about 160 pounds or less. A smaller female patient would weigh about 130 pounds or less. Patients having a Body Mass Index of 30 and higher is considered obese.

In addition, older patients may need a lower dose at initiation, with a gradual increase to the recommended dose after days or weeks. In another embodiment, older patients may need lower doses for the duration of treatment. The aged population includes the "young old" who are 65-74, the "old old" who are 75-84 and the "frail elderly" who are 85 and older. For example, a starting dose of 30 mg per day for two weeks, followed by 60 mg per day for 4 weeks, then by 90 mg per day. Titration may even be warranted up to about 120 mg per day.

Another embodiment involves the treatment with 60-120 mg of Fasudil hydrochloride hemihydrate once per day in an extended release dosage form. Treatment with an extended release total daily dose of 90 mg Fasudil hydrochloride hemihydrate is preferred.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

The treatment methods of the present invention, while contemplating various routes of administration, are particularly suited to oral administration. Thus, it will be understood that an effective amount of the ROCK inhibitor or a pharmaceutically acceptable salt thereof preferably is administered orally one or more times orally per day and an effective amount may range from about 10 mg to about 1000 mg. Fasudil hydrochloride hemihydrate, for example, is suitably administered in a daily oral amount of about 10 mg to about 500 mg, about 10 mg to about 400 mg, about 10 mg to about 200 mg, about 10 mg to about 100 mg, about 20 mg to about 10 mg. One preferred dosing regimen involves the treatment with 20, 30 or 40 mg of Fasudil hydrochloride hemihydrate orally three times per day using an immediate-release formulation, for a total daily dose of 60 - 120 mg. Most preferred dosing exceeds a daily oral dose of 60 mg, with most preferred ranges for daily oral dosing being 70 mg to 120 mg administered in three equal amounts during the day. Other preferred daily oral doses will range from 90 mg to 120 mg per day or 80 mg to 140 mg per day. A further oral dosing regimen involves the treatment with, 30 to 60 mg of Fasudil hydrochloride hemihydrate only two times per day using an immediate-release formulation, for a total daily oral dose of 60 - 120 mg. Based on ROCK inhibitory activity, one skilled in the art can readily extrapolate the provided dosing ranges for fasudil to other ROCK inhibitors.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

Methods of administering compositions according to the invention would generally be continued for at least one day. Some preferred methods treat for up to 30 days or up to 60 days or even up to 90 days or even more. Treatment for more than 60 days is preferred and treatment for at least 6 months is particularly preferred. Some embodiments contemplate treatment for 12 months or 18 months or more. The precise duration of treatment will depend on the patient's condition and response to treatment.

In one embodiment treatment of an FTD patient with fasudil reduces at least one symptom in the patient by at least 10%; 15%;20%; 25%; 30%; 35%; 40%; 45% or 50%. In one embodiment, the symptom reduced (improved) is apathy, lack of emotional concern/loss of empathy, hyperorality or hyperphagia, and stereotypic behavior. In another embodiment, treatment with fasudil reduces number of incidents of social misconduct including disinhibition, impaired judgment, ritualized or repetitive behavior, and excess sleeping by at least 10%; 15%;20%; 25%; 30%; 35%; 40%; 45% or 50%. In one specific embodiment, bvFLTD patients treated with fasudil will exhibit improvements in deficits in frontal or executive tasks and marked behavior and personality changes and/or a slowing in decline vs. control patients following commencement of treatment. In another specific embodiment, improvement of bvFLTD patients can be assessed by increased scores on the scales that measure social behavior such as the Social Cognition and Emotional Assessment (SEA), the Frontotemporal Dementia Rating Scale (FRS), the Frontal Systems Behavior Scale (FrSbe) and the FTLD Clinical Dementia Rating scale.

In one embodiment, the scores will increase by at least 0.5 to 1 point (where applicable), preferably by at least 2 points, more preferably by at least 3 points and most preferably at least 5 points.

In another specific embodiment, SD patients will generally show improvements in deficits in comprehension and naming and/or a slowing in decline vs. control following commencement of treatment.

PNFA patients will generally show improvements in deficits in motor speech and grammatical sentence construction and/or a slowing in decline vs. control following commencement of treatment.

In addition to the specific symptoms, treatment using fasudil will generally result in improved cognitive functioning. Patients will generally show improvement on the MMSE and/or the SIB and preferentially of at least 2 points during the early stages of treatment and declines in cognition are slowed relative to control patients. Other short tools for assessing dementia/diminished cognition and for measuring cognitive improvement include: the Eight-item Informant Interview to Differentiate Aging and Dementia (AD8); the Annual Wellness Visit (AWV); the General Practitioner Assessment of Cognition (GPCOG); Health Risk Assessment (HRA); Memory Impairment Screen (MIS); the Montreal Cognitive Assessment (MoCA); the St. Louis University Mental Status Exam (SLUMS); and the Short Informant Questionnaire on Cognitive Decline in the Elderly (Short IQCODE).

The methods of the invention also contemplate administering the ROCK inhibitor with other compounds used to treat other forms of dementia or used to treat symptoms of FTD. They may be administered in combination, a single dosage form, in a common dosing regimen or administered to the same patient at different times of the day using different dosing regimens. For example, drugs are included that are used to treat cognition and/or pseudobulbar affect.

Two classes of drugs are used to treat dementia other than FTD and have been shown to improve cognition: acetylcholinesterase inhibitors and N-methyl-D-aspartate (NMDA) receptor antagonists. Generally used in the early stages of disease, acetylcholinesterase inhibitors prevent the breakdown of the neurotransmitter acetylcholine. These drugs include piperidines like donepezil (Aricept), phenanthrene derivatives, like galantamine (Razadyne), and carbamates like rivastigmine (Exelon). NMDA receptor antagonists include the uncompetetitive inhibitor memantine (Namenda). A combination of memantine and donepezil (Namzaric) is also available. However, these drugs have not been found useful for FTD and/or have even worsened cognitive function (memantine) in open-label or placebo-controlled trials, illustrating the limitations of extrapolating efficacy of drugs for FTD based on other neurodegenerative diseases or other types of dementia (Tsai and Boxer 2014). FTD patients with motor deficits are generally not responsive to dopamine used to treat Parkinson's disease, either, although dopamine reuptake inhibitors have been shown to improve behavioral symptoms such as risk-taking in FTD patients

In one embodiment, the patient is administered fasudil in combination with levodopa or a dopamine agonist, including but not limited to pramiprexole, ropinirole, apomorphine, and rotigotine. In a specific embodiment, the levodopa is administered in a dose of from about 30 to 2500 mg per day. In a further specific embodiment, the dopamine agonist is administered in a dose of from 0.25 to 10 mg per day. In another embodiment, fasudil is administered in combination with amantadine. In a specific embodiment, amantadine is administered in a dose of about 100-400 mg per day.

SSRIs are generally used to manage symptoms of FTD. The invention contemplates use of fasudil in combination with antidepressants, including SSRIs such as citalopram or escitalopram, paroxetine, fluoxetine, or sertraline. In one embodiment, the antidepressant is administered in a dose of about 10-40 mg/day.

Dextromethorphan hydrobromide is another an uncompetitive NMDA receptor antagonist that also has activity as a sigma-1 receptor agonist and is contemplated for use with fasudil for the treatment of FTD. Marketed in combination quinidine sulfate (a CYP450 2D6 inhibitor), the product Nudexta is indicated for the treatment of pseudobulbar affect, which occurs in many forms of dementia.

### LIST OF REFERENCES

Ahmed RM, Irish M, Henning E, Dermody N, Bartley L, Kiernan MC, Piguet O, Farooqi S, Hodges JR. Assessment of Eating Behavior Disturbance and Associated Neural Networks in Frontotemporal Dementia. AMA Neurol. 2016 Mar;73(3):282-90.
Benussi A, Di Lorenzo F, Dell'Era V,, Cosseddu M, Alberici A, Caratozzolo S, Cotelli MS, Micheli A, Rozzini L, Depari A, Flammini A, Ponzo V, Martorana A, Caltagirone C, Padovani A, Koch G, Borroni B, Transcranial magnetic stimulation distinguishes Alzheimer disease from frontotemporal dementia. Neurology. 2017; 89*7):665-672.
Bonner M, Ash S, and Grossman M, Curr Neurol Neurosci Rep. The New Classification of Primary Progressive Aphasia into Semantic, Logopenic, or Nonfluent/Agrammatic Variants. 10(6): 484-490 (2010).
Burrell JR, Yiannikas C, Rowe D, Kiernan MC, Predicting a Positive Response to Intravenous Immunoglobulin in Isolated Lower Motor Neuron Syndromes. PLos One. 2011; 6(10): e27041.
Connor DJ, Sabbagh MN, Cummings JL. Comment on administration and scoring of the Neuropsychiatric Inventory (NPI) in clinical trials. Alzheimers Dement. 2008 Nov; 4(6): 390-394.
Feng Y. LoGrasso P, Defert O, Li R, Rho Kinase (ROCK) Inhibitors and Their Therapeutic Potential. J Med Chem. 2016, 59*6): 2269-2300.
Folstein MF, Folstein SE, McHugh PR. "Mini-mental state": a practical method for grading the cognitive state of patients for the clinician. J Psychiatr Res. 1975;12:189-198.
Funkiewiez, A., Bertoux, M., de Souza, L. C., Levy, R., & Dubois, B. The SEA (Social Cognition and Emotional Assessment): A Clinical Neuropsychological Tool for Early Diagnosis of Frontal Variant of Frontotemporal Lobar Degeneration. Neuropsychology. Neuropsychology. 2012; 26(1): 81-90.
Jacobs M, Hayakawa K, Swenson L, Bellon S, Fleming M, Taslimi P, Doran J, The structure of dimeric ROCK I reveals the mechanism for ligand selectivity. J Biol Chem. 2006; 281(1): 260-68.
Kamei S, Oishi M, Takasu T. 1996a. Evaluation of fasudil hydrochloride treatment for wandering symptoms in cerebrovascular dementia with 31P-magnetic resonance spectroscopy and Xe-computed tomography. Clin Neuropharmacol. 19:428-38.
Kamei S, Toshiaki T, Oishi M, Effect of fasudil hydrochloride on wandering symptoms of c cerebrovascular dementia patients. Neurotherapy. 1996b 13:43-50.
Kramer JH, Mungas D, Possin KL, Rankin KP, Boxer AL, Rosen HJ, Bostrom A, Sinha L, Berhel A, Widmeyer M. NIH EXAMINER: Conceptualization and Development of an Executive Function Battery. J Int Neuropsychol Soc. 2014; 20: 11-19.
Mesulam M, Wieneke C, Rogalski E, et al. Quantitative template for subtyping primary progressive aphasia. Arch Neurol. 2009;66:1545-1551.
Mioshi E, Hsieh S, Savage S, Hornberger M, and Hodges J, Clinical staging and disease progression in frontotemporal dementia. Neurology 74 (2010).
Nakagawa O, Fukisawa K, Ishizaki T, Saito Y, Nakao K, Narumiya S, ROCK-I and ROCK-II, two isoforms of Rho-associated coiled-coil forming protein serine/threonine kinase in mice. FEBS Lett. 1996 Aug 26;392(2):189-93.
Rovner BW, Folstein MF. Mini-mental state exam in clinical practice. Hosp Pract. 1987;22(1A):99, 103, 106, 110.
Shibuya M, Asano T, Sasaki Y. 2001. Effect of Fasudil HCl, a protein kinase inhibitor, on cerebral vasospasm. Acta Neurochir Suppl. 77:201-4.
Tsai and Boxer, Curr Treat Options Neurol. Treatment of Frontotemporal Dementia, 16(11): 319 (2014).
Uehata M, Ishizaki T, Satoh H, Ono T, Kawahara T, Morishita T, Tamakawa H, Yamagami K, Maekawa M, Narumiya S, Calcium sensitization of smooth muscle mediated by a Rho-associated protein kinase in hypertension. Nature. 1997 Oct 30;389(6654):990-4.
Yamaguchi H, Miwa Y, Kasa M, Kitano K, Amano M, Kaibuchi K, Hakoshima T, Structural basis for induced-fit binding of Rho-kinase to the inhibitor Y-27632. J Biochem. 2006 Sep;140(3):305-11.

## Claims

1. A pharmacologically effective amount of fasudil or a pharmaceutically acceptable salt thereof for use in the treatment of behavioral variant frontotemporal dementia (bvFTD), progressive non-fluent (agrammatic) aphasia (PNFA) or semantic dementia (SD) in patient, wherein the patient has not been diagnosed with amyotrophic lateral sclerosis, corticobasal degeneration, Parkinson's Disease or progressive supranuclear palsy.

2. The pharmacologically effective amount of fasudil or a pharmaceutically acceptable salt thereof for use according to claim 1, wherein said treatment occurs only after the onset of symptoms.

3. The pharmacologically effective amount of fasudil or a pharmaceutically acceptable salt thereof for use according to claim 1 or 2, wherein the patient is suffering from the behavioral variant frontotemporal dementia.

4. The pharmacologically effective amount of fasudil or a pharmaceutically acceptable salt thereof for use according to any one of claims 1 to 3, wherein the patient has no neuromuscular symptoms.

5. The pharmacologically effective amount of fasudil or a pharmaceutically acceptable salt thereof for use according to any one of claims 1 to 4, wherein fasudil is administered orally in a total daily dose of between 70 mg and 140 mg.

6. The pharmacologically effective amount of fasudil or a pharmaceutically acceptable salt thereof for use of claim 5 wherein the total daily dose is 90 mg.

7. The pharmacologically effective amount of fasudil or a pharmaceutically acceptable salt thereof for use according to any one of claims 1 to 6, wherein said pharmaceutically acceptable salt thereof is fasudil hydrochloride hemihydrate.

## Patentansprüche

1. Eine pharmakologisch wirksame Menge von Fasudil oder eines pharmazeutisch verträglichen Salzes davon zur Verwendung bei der Behandlung der Verhaltensvariante der frontotemporalen Demenz (bvFTD), der progressiven nichtflüssigen (agrammatischen) Aphasie (PNFA) oder der semantischen Demenz (SD) bei einem Patienten, wobei der Patient nicht mit amyotropher Lateralsklerose, kortikobasaler Degeneration, Parkinson-Krankheit oder progressiver supranukleärer Blickparese diagnostiziert worden ist.

2. Die pharmakologisch wirksame Menge von Fasudil oder eines pharmazeutisch verträglichen Salzes davon zur Verwendung gemäß Anspruch 1, wobei die Behandlung nur nach Auftreten der Symptome erfolgt.

3. Die pharmakologisch wirksame Menge von Fasudil oder eines pharmazeutisch verträglichen Salzes davon zur Verwendung gemäß Anspruch 1 oder 2, wobei der Patient an der Verhaltensvariante der frontotemporalen Demenz leidet.

4. Die pharmakologisch wirksame Menge von Fasudil oder eines pharmazeutisch verträglichen Salzes davon zur Verwendung gemäß einem der Ansprüche 1 bis 3, wobei der Patient keine neuromuskulären Symptome aufweist.

5. Die pharmakologisch wirksame Menge von Fasudil oder eines pharmazeutisch verträglichen Salzes davon zur Verwendung gemäß einem der Ansprüche 1 bis 4, wobei Fasudil oral in einer Gesamttagesdosis von 70 mg bis 140 mg verabreicht wird.

6. Die pharmakologisch wirksame Menge von Fasudil oder eines pharmazeutisch verträglichen Salzes davon zur Verwendung gemäß Anspruch 5, wobei die Gesamttagesdosis 90 mg beträgt.

7. Die pharmakologisch wirksame Menge von Fasudil oder eines pharmazeutisch verträglichen Salzes davon zur Verwendung gemäß einem der Ansprüche 1 bis 6, wobei es sich bei dem pharmazeutisch verträglichen Salz um Fasudilhydrochlorid-Hemihydrat handelt.

## Revendications

1. Une quantité pharmacologiquement efficace de fasudil ou d'un sel pharmaceutiquement acceptable de celui-ci pour une utilisation dans le traitement de la démence frontotemporale variant comportemental (bvFTD), de l'aphasie progressive non fluente (agrammatique) (PNFA) ou de la démence sémantique (SD) chez un patient, ledit patient n'ayant pas été diagnostiqué comme atteint de sclérose latérale amyotrophique, de dégénérescence corticobasale, de la maladie de Parkinson ou de paralysie supranucléaire progressive.

2. La quantité pharmacologiquement efficace de fasudil ou d'un sel pharmaceutiquement acceptable de celui-ci pour une utilisation selon la revendication 1, ladite administration n'ayant lieu qu'après l'apparition des symptômes.

3. La quantité pharmacologiquement efficace de fasudil ou d'un sel pharmaceutiquement acceptable de celui-ci pour une utilisation selon la revendication 1 ou 2, le patient souffrant de la démence frontotemporale variant comportemental.

4. La quantité pharmacologiquement efficace de fasudil ou d'un sel pharmaceutiquement acceptable de celui-ci pour une utilisation selon l'une quelconque des revendications 1 à 3, le patient ne présentant pas de symptômes neuromusculaires.

5. La quantité pharmacologiquement efficace de fasudil ou d'un sel pharmaceutiquement acceptable de celui-ci pour une utilisation selon l'une quelconque des revendications 1 à 4, le fasudil étant administré par voie orale à une dose journalière totale comprise entre 70 mg et 140 mg.

6. La quantité pharmacologiquement efficace de fasudil ou d'un sel pharmaceutiquement acceptable de celui-ci pour une utilisation selon la revendication 5, la dose journalière totale étant de 90 mg.

7. La quantité pharmacologiquement efficace de fasudil ou d'un sel pharmaceutiquement acceptable de celui-ci pour une utilisation selon l'une quelconque des revendications 1 à 6, ledit sel pharmaceutiquement acceptable étant le chlorhydrate d'hémihydrate de fasudil.
